(19) Europäisches Patentamt
European Patent Office
Office européen des brevets

(11) Veröffentlichungsnummer: **0 157 361**
**B1**

# (12) EUROPÄISCHE PATENTSCHRIFT

(45) Veröffentlichungstag der Patentschrift: **23.08.89**

(51) Int. Cl.⁴: **C 12 Q 1/34**

(21) Anmeldenummer: **85103668.1**

(22) Anmeldetag: **27.03.85**

## (54) Analysenverfahren und Mittel zum Nachweis esterolytischer und/oder proteolytischer Enzyme.

(30) Priorität: **06.04.84 DE 3413119**

(43) Veröffentlichungstag der Anmeldung: **09.10.85 Patentblatt 85/41**

(45) Bekanntmachung des Hinweises auf die Patenterteilung: **23.08.89 Patentblatt 89/34**

(84) Benannte Vertragsstaaten: **DE FR GB IT NL SE**

(56) Entgegenhaltungen:
**EP-A-0 014 929**
**EP-A-0 034 323**
**EP-A-0 094 554**

**CHEMICAL ABSTRACTS, Band 100, Nr. 9, 27. Februar 1984, Seite 249, Zusammenfassung Nr. 64007y, Columbus, Ohio, US; T. ABE et al.: "Influence of long-chain primary alcohols on cholesterol exidase from Schizophyllum commune", & BIOCHIM. BIOPHYS. ACTA 1983, 749(1), 69-76**

(73) Patentinhaber: **MILES INC., 1127 Myrtle Street, Elkhart Indiana 46514 (US)**

(72) Erfinder: **Schnabel, Eugen, Dr., Schimmelweg 6, D-5600 Wuppertal 11 (DE)**

(74) Vertreter: **Dänner, Klaus, Dr., c/o Bayer AG Konzernverwaltung RP Patentabteilung, D-5090 Leverkusen 1 Bayerwerk (DE)**

**EP 0 157 361 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Mittel für den analytischen Nachweis von esterolytischen und/oder proteolytischen Enzymen, z. B. in Körperflüssigkeiten, wobei die Ester in geeigneter Weise in Testmitteln, insbesondere Teststreifen, inkorporiert sind. Die erfindungsgemäßen Mittel enthalten neben chromogenen Enzymsubstraten (Aminosäure- oder Peptidestern von geeigneten Phenolen) sowie gegebenenfalls mit den Phenolen unter Farbbildung kuppelnden Diazoniumsalzen noch Undecanol als Beschleuniger für die enzymatische Spaltung der Aminosäure- bzw. Peptidester. Bevorzugt werden die Mittel für den Nachweis von Leukozyten, insbesondere im Urin, eingesetzt.

In der Diagnostik der Erkrankungen der Nieren und des Urogenitaltraktes hat der Nachweis von Leukozyten in Körperflüssigkeiten, insbesondere im Urin, große Bedeutung. Ursprünglich erfolgte dieser Nachweis durch Auszählen der Leukozyten im nicht zentrifugierten Harn oder im Harnsediment. Mit beiden Methoden können nur intakte Leukozyten erfaßt werden. Es ist jedoch bekannt, daß die Geschwindigkeit der Leukozyten-Lyse je nach Harnmilieu starken Schwankungen unterworfen ist; so beträgt z. B. in stark alkalischen Harnen die Leukozyten-Halbwertszeit nur 60 Minuten. Dies führt dazu, daß zu niedrige Leukozytenzahlen festgestellt werden. Von diesem Lyse-Fehler abgesehen, liefert die quantitative mikroskopische Bestimmung der Leukozyten im nicht zentrifugierten, homogenisierten Harn in der Zählkammer sehr genaue Werte. Trotzdem wird diese Methode in der Praxis nur selten angewandt, da sie mühevoll und zeitraubend ist und geschultes Personal voraussetzt.

Das in der medizinischen Praxis bevorzugte Verfahren für die Leukozytenbestimmungen in Harn war daher die sogenannte Gesichtsfeldmethode im Harnsediment. Hierzu mußte zunächst die Probe (Sediment) durch Zentrifugieren gewonnen werden. Dabei wurden jedoch auch andere Bestandteile des Harnes angereichert, die - wie z. B. Salze und Epithelzellen - die mikroskopische Auszählung der Leukozyten beträchtlich erschweren. Schwankender Sedimentgehalt, Inhomogenitäten des Sediments sowie unterschiedliche optische Ausstattung der Mikroskope führten zu relativ großen Fehlern (bis zu mehreren hundert Prozent) bei der Angabe der Leukozytenzahl.

Um diese Schwierigkeiten zu vermeiden, wurde bereits vielfach versucht, als Nachweisprinzip für Leukozyten in verschiedenen Körperflüssigkeiten enzymatische Reaktionen heranzuziehen, da die Leukozyten ein breitgefächertes Enzymspektrum besitzen.

So sind z. B. aus den deutschen Offenlegungsschriften 2 826 965 und 2 836 644 Mittel zum Nachweis von Leukozyten in Körperflüssigkeiten bekannt, bei denen die in Leukozyten vorhandene esterolytische und/oder proteolytische Aktivität für analytische Zwecke ausgenutzt wird. Als Substrate für die Leukozyten-Esterasen und/oder -Proteasen werden dabei Sulfonphthaleinester bzw. Azo-Farbstoffester verwendet. Die bei der enzymatischen Umsetzung freigesetzten Farbstoffe werden dann nach bekannten Methoden bestimmt. Die in diesen Publikationen beschriebenen Mittel sind jedoch für praktische Zwecke noch zu unempfindlich, da sie bei niedrigen Leukozyten-Konzentrationen zu lange Reaktionszeiten aufweisen.

Verschiedene Methoden für den Nachweis von Proteasen und Esterasen sind auch aus der histo- und cytochemischen Enzymologie bekannt (vgl. beispielsweise A.G.E. Pearse, Histochemistry, Theoretical and Applied, 3. Ed., Churchill Livingstone, Edinburgh-London-New York 1968). Zum Nachweis werden dabei im allgemeinen farblose oder schwach gefärbte Ester eingesetzt, die durch die Enzyme in eine farblose Säure und eine ebenfalls farblose Alkohol - (Phenol) -Komponente gespalten werden. Die Phenolkomponente wird dann in einer Folgereaktion zu farbigen Produkten umgesetzt, z. B. durch Kupplung mit Diazoniumsalzen oder durch Oxidation. F. Schmalzl und H. Braunsteiner beschreiben zum Beispiel in Klin. Wschr. 46, 642 (1968) einen spezifischen cytochemischen Leukozytenesterasenachweis mit Naphthol-AS-D-Chloracetat als Substrat und einem Diazoniumsalz, das mit dem freiwerdenden Naphthol eine farbige Azo-Verbindung bildet.

Für den schnellen und einfachen Nachweis von Leukozyten in Körperflüssigkeiten, wie z. B. im Harn, erwiesen sich Zweikomponenten-Systeme dieser Art jedoch als nicht geeignet, da sie viel zu unempfindlich sind: Proben mit 5000 Leukozyten/µl zeigen noch keine Reaktion.

In GB-A-1 128 371 und EP-A-12 957 wird die Verwendung von Indoxyl- und Thioindoxylestern als chromogenen Substraten für den Nachweis von hydrolytischen Enzymen in Körperflüssigkeiten beschrieben. Bei der enzymatischen Spaltung des Substrats entsteht freies Indoxyl, welches anschließend zum leicht nachweisbaren blauen Farbstoff Indigo oxidiert wird. Ein handelsüblicher Test auf Basis von EP-A-12 957 besteht aus einem Streifen Filterpapier, welches mit dem N-Tosyl-L-alanin-indoxylester imprägniert ist. Beim Eintauchen in eine Leukozyten enthaltende Urinprobe färbt sich der Teststreifen blau. Ein wesentlicher Nachteil dieses Produkts ist jedoch die lange Wartezeit (ca. 15 Minuten), bis die Endfärbung erreicht ist und der Test ausgewertet werden kann.

In EP-A-14 929 werden verschiedenartige Beschleuniger (Pyridinderivate; Imidazolderivate; Alkohole; Metallkomplexe) für die enzymatische Spaltungsreaktion beschrieben. Nachteilig bleiben jedoch die relativ lange Zeit bis zur vollständigen Oxidation des Indoxyls und die geringe Empfindlichkeit des Tests (Nachweisgrenze: einige Tausend Leukozyten/µl). Gleiches trifft auch für die Verwendung der Ester von Leuko-Indoanilinen als Substrate für Leukozyten-Enzyme gemäß EP-A-34 323 zu.

EP-A-39 880 stellt eine Kombination der Substrate gemäß EP-A-12 957 bzw. 14 929 mit den weiter oben diskutierten Nachweisprinzip der Kupplung mit Diazoniumsalzen dar. Es gelingt auf diese Weise zwar, die Erfassungsgrenzen für Leukozyten deutlich zu senken, jedoch wird die für die Praxisanwendung gewünschte Nachweisempfindlichkeit von 15 - 20 Leukozyten/µl noch nicht erreicht.

Aufgabe der vorliegenden Erfindung war es daher, neue Beschleuniger für esterspaltende Enzyme aufzufinden, die infolge einer Beschleunigung der enzymatischen Spaltung der Substrate durch die Leukozytenenzyme einen empfindlichen und rascheren Nachweis der Leukozyten in Harn ermöglichen. Es wurde nun überraschend gefunden, daß n-Undecanol die Leukozyten-Enzyme stark aktiviert. Aus EP-A-14 929 sind Alkohole als potente Aktivatoren der Leukozytenenzyme bekannt geworden, die die Spaltung von Arylestern gemäß EP-A-7 404, EP-A-8 428 und EP-A-12 957 beträchtlich beschleunigen. Ebenso wird die enzymatische Spaltung der bereits früher beschriebenen Substrate [G.Gomori, J.Histochem. Cytochem. 6, 469 (1953); H. Löffler, Klin.-Wochenschr. 39, 1120 (1961) sowie L. Visser und E. Blout, Fed.Proc. 28, 407 (1969) und Biochim. Biophys. Acta 268, 257 (1972) und F. Sweetman und L Ornstein, J.Histochen. Cytochem. 23, 327 (1974)] durch Alkohole stark aktiviert.

In EP-A-14 921 werden Alkohole der allgemeinen Formel R-OH als Aktivatoren genannt, mit der Maßgabe, daß R ein Alkylrest ist.

Als Beispiele werden in dieser Anmeldung Hexanol, Heptanol, Octanol, Nonanol, Decanol, Dodecanol, Tridecanol usw. aufgeführt. Es wurde nun überraschend gefunden, daß das in EP-A-14 929 nicht erwähnte n-Undecanol alle vorgenannten Alkohole in der Aktivierung der Leukozyten-Enzyme übertrifft.

Das Undecanol wird im Flüssigtest bevorzugt in Konzentrationen von $10^{-4}$ bis 1 Gew.-% eingesetzt, vorzugsweise von $10^{-3}$ bis 0,1 Gew.-% (jeweils bezogen auf Testlösung). Bei der unten beschriebenen Herstellung von Testvorrichtungen wird das Undecanol den Imprägnierlösungen in Konzentrationen von 0,01 bis 10 Gew.-%, vorzugsweise 0,5 bis 5 Gew.-% zugesetzt.

Gegenstand der Erfindung sind Mittel zum Nachweis esterolytischer und/oder proteolytischer Enzyme, enthaltend

(a) einen Aminosäure- oder Peptidester eines Phenols als chromogenes Enzymsubstrat,
(b) einen Alkohol als die enzymatische Spaltung der Aminosäure- bzw. Peptidesterbindung von Komponente (a) beschleunigende Substanz, gegebenenfalls
(c) ein Diazoniumsalz, gegebenenfalls
(d) einen Puffer, sowie gegebenenfalls
(e) ein Trägermaterial und/oder übliche Zusatzstoffe,

welche dadurch gekennzeichnet sind, daß als beschleunigende Substanz n-Undecanol eingesetzt wird.

Gegenstand der Erfindung ist schließlich auch ein Verfahren zum Nachweis von esterolytischen und/oder proteolytischen Enzymen in flüssigen Proben, insbesondere Körperflüssigkeiten, welches dadurch gekennzeichnet ist, daß man die Probe mit den erfindungsgemäßen Mittel in Kontakt bringt und die auftretende Farbreaktion bestimmt.

Das erfindungsgemäß zu verwendende n-Undecanol beschleunigt die enzymatische Spaltung der in den EP-A-7 404, 8 428, 12 957, 14 929, 34 323 und 39 880 beschriebenen Substrate durch die Leukozytenenzyme ebenso wie die Spaltung der bereits früher beschriebenen Substrate [G.Gomori, J.Histochem. Cytochem. 6 469 (1953); H. Löffler, Klin. Wochenschr. 39, 1120 (1961); L. Visser und E. Blout, Fed-Proc. 28, 407 (1969) sowie Biochim. Biophys. Acta 268, 257 (1972) und F. Sweetman und L. Ornstein, J. Histochem. Cytochem. 23, 327 (1974)].

Zu den bevorzugten chromogenen Substraten in den erfindungsgemäßen Mitteln gehören auch die in einer parallelen Anmeldung beschriebenen Verbindungen der allgemeinen Formel

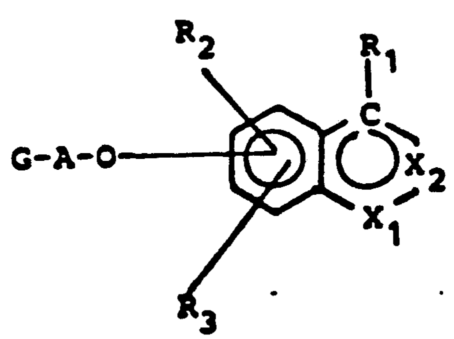

(II)

in welcher

$X_1$ und $X_2$ gleich oder verschieden sind und Stickstoff oder Schwefel bedeuten, mit der Maßgabe, daß $X_1$ und $X_2$ nicht gleichzeitig für Schwefel stehen;

$R_1$ für Wasserstoff oder eine gegebenenfalls verzweigte Alkylgruppe mit 1 bis 6 C-Atomen steht, die gegebenenfalls durch Halogen oder Hydroxy substituiert sein kann;

$R_2$ und $R_3$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppen stehen, die ihrerseits wieder durch $C_1$-$C_6$-Alkyl-

gruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_2$ und $R_3$ gemeinsam einen annellierten aromatischen Ring, vorzugsweise einen Benzolring, bilden, welcher seinerseits mit einem oder zwei Resten $R_2$ substituiert sein kann;

A einen Aminosäure- oder Peptidrest bedeutet und
G Wasserstoff oder vorzugsweise eine in der Peptidchemie übliche oder davon abgeleitete Stickstoffschutzgruppe darstellt.

Bevorzugte Verbindungen der allgemeinen Formel (II) sind solche, bei denen $X_1$ für Schwefel und $X_2$ für Stickstoff stehen. Bevorzugt sind weiterhin Verbindungen der Formel (II), in denen $R_1$ für Wasserstoff steht und solche, bei denen $R_2$ und $R_3$, die gleich oder verschieden sind, für Wasserstoff, $C_1$-$C_2$-Alkyl, $C_1$-$C_2$-Alkoxy, Halogen, $C_1$-$C_4$-Dialkylaminogruppen oder Benzolreste stehen.

Besonders bevorzugt steht bei den Verbindungen von Formel (II) der Esterrest in 5-Position.

Weitere erfindungsgemäß bevorzugte chromogene Substrate sind die ebenfalls in einer parallelen Anmeldung beschriebenen Verbindungen der allgemeinen Formel

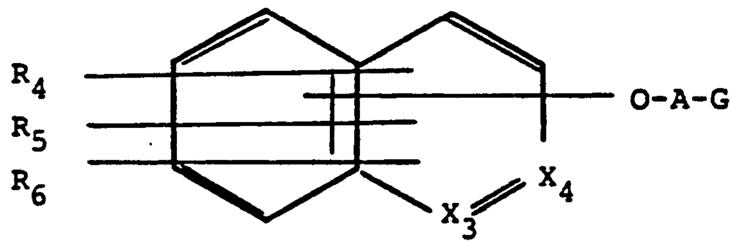

(III)

in welcher
$X_3$ und $X_4$ für N oder CH stehen, mit der Maßgabe, daß jeweils entweder $X_3$ oder $X_4$ N für steht,
$R_4$, $R_5$ und $R_6$ gleich oder verschieden sind und für Wasserstoff, $C_1$-$C_6$-Alkylgruppen, $C_1$-$C_6$-Alkoxygruppen, $C_1$-$C_6$-Acylgruppen, Halogen, Trifluormethyl, Nitro, $SO_3H$, Cyano, $C_1$-$C_8$-Acylaminogruppen, $C_1$-$C_6$-Dialkylaminogruppen oder $C_6$-$C_{10}$-Arylgruppenstehen, die ihrerseits wieder durch $C_1$-$C_6$-Alkylgruppen, $C_1$-$C_6$-Alkoxygruppen, Halogen, Cyano, Nitro, Trifluormethyl, $SO_3H$, $C_1$-$C_6$-Acylgruppen oder $C_1$-$C_6$-Dialkylaminogruppen substituiert sein können, oder $R_5$ und $R_6$ gemeinsam einen annellierten aromatischen Ring, vorzugsweise einen Benzolring, bilden, welcher seinerseits mit einem oder zwei Resten $R_4$ substituiert sein kann,

und A und G die oben bei Formel (II) angegebene Bedeutung haben.

In den Verbindungen gemäß allgemeiner Formel (III) steht vorzugsweise $X_3$ für CH und $X_4$ für Stickstoff. Bevorzugt bedeuten $R_4$, $R_5$ und $R_6$, die gleich oder verschieden sein können, für Wasserstoff, $C_1$-$C_4$-Alkyl, $C_1$-$C_4$-Alkoxy, Acylamino (wobei der Säurerest aliphatisch oder aromatisch mit 1 - 6 C-Atomen sein kann), $C_1$-$C_4$-Dialkylamino, Nitro, Cyano, Halogen sowie Aryl, das gegebenenfalls substituiert ist durch $C_1$-$C_4$-Alkyl, $C_1$-$C_4$-Alkoxy oder Halogen. Besonders bevorzugt sind $R_4$, $R_5$ und $R_6$ Wasserstoff, $C_1$-$C_4$-Alkyl, Phenyl oder Halogen oder $R_5$ und $R_6$ bilden zusammen einen annellierten Benzolring.

Als chromogene Substrate für die erfindungsgemäßen Mittel eignen sich darüber hinaus auch Verbindungen der allgemeinen Formel

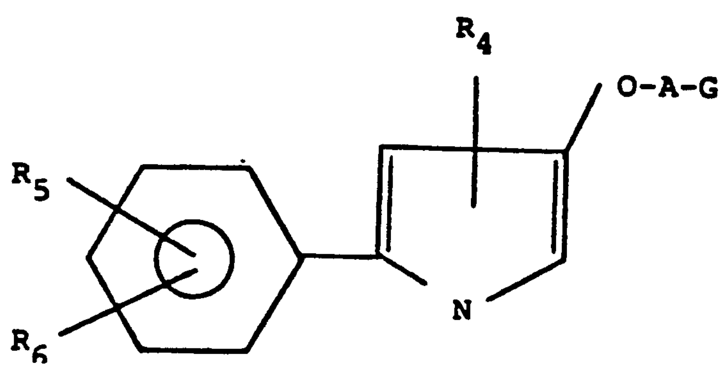

(IV)

wobei $R_4$, $R_5$, $R_{61}$ A und G die oben bei Formel (III) angegebene Bedeutung haben.

In den allgemeinen Formeln (II), (III) und (IV) steht % G - A - bevorzugt für einen Rest der allgemeinen Formel

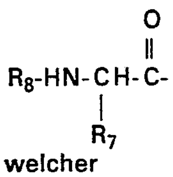

in welcher

$R_7$ für Wasserstoff oder einen gegebenenfalls verzweigten Alkyl-, Cycloalkyl- oder Arylrest mit 1 - 15 C-Atomen, bevorzugt 1 - 9 C-Atomen, steht, welcher gegebenenfalls durch eine Hydroxy-, eine Mercapto- oder eine Carboxylgruppe substituiert ist, und

$R_8$ Wasserstoff oder vorzugsweise -CO-Alkyl, -CO-Aralkyl, -CO-Aryl, $-SO_2$-Alkyl oder $-SO_2$-Aryl darstellt, wobei Alkylreste geradkettig oder verzweigt mit 1 - 9 C-Atomen, bevorzugt 1 - 6 C-Atomen sind und Arylreste bevorzugt Benzolringe, die gegebenenfalls durch $C_1$-$C_4$-Alkylgruppen, $C_1$-$C_4$-Alkoxygruppen oder Halogen substituiert sind, darstellen.

Besonders bevorzugt steht G-A- für einen mit einer üblichen Stickstoffschutzgruppe versehenen Rest einer natürlichen Aminosäure oder eines Peptids aus 2 bis 8 solcher Aminosäuren.

Die Aminosäurereste können dabei in ihrer L- oder D-Form oder auch in ihrer racemischen Form vorliegen. Besonders bevorzugt sind die Reste von Glycin, Alanin, Valin, Leucin, Isoleucin, Phenylalanin und Tyrosin, wobei jeweils die L-Form besonders bevorzugt ist. Gegebenenfalls vorhandene freie Hydroxygruppen können acyliert, vorzugsweise acetyliert, sein.

Unter einem Peptidrest in der Definition von A sind z. B. Di-, Tri-, Tetra- und Pentapeptide, vorzugsweise Di- und Tripeptide, zu verstehen, wobei als Aminosäure-Komponenten vorzugsweise die oben erwähnten Aminosäuren in Betracht kommen.

Die Substrate der allgemeinen Formeln (II), (III) und (IV) werden erhalten, indem man die entsprechenden Phenole mit Aminosäuren bzw. Peptiden der allgemeinen Formel

G - A - OH

in welcher G und A die oben angegebene Bedeutung haben, bzw. geeigneten reaktiven Derivaten davon nach in der Peptidchemie üblichen Methoden umsetzt.

Geeignete reaktive Derivate sind z. B. die Säurechloride und die bei der Peptidsynthese üblicherweise verwendeten Mischanhydride, z. B. mit Chlorameisensäureethylester, oder Aktivester wie z. B. Pentachlorphenylester oder N-Hydroxybenztriazolester.

Die erfindungsgemäßen Mittel enthalten vorzugsweise als mit den (bei der enzymatischen Spaltung freigesetzten) Phenolen reagierende Farbbildner Diazoniumsalze der allgemeinen Formel

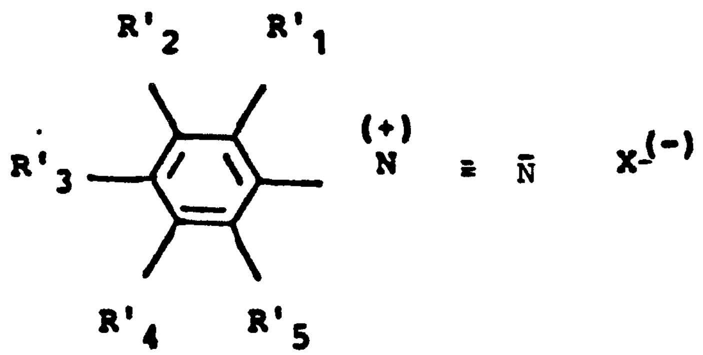 (V)

in der

$R'_1$ eine niedere Alkyl-, eine niedere Alkoxy-, eine niedere Alkylmercapto-, eine Hydroxy-, Nitro-, Cyano-, Trifluormethyl-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino, eine N-Thiomorpholino-, eine N-Pyrrolidino-, eine gegebenenfalls N'-alkylierte N-Piperazino-, eine N-Piperidino-Gruppe, Halogen oder Wasserstoff,

$R'_3$ eine niedere Alkyl-, eine niedere Alkoxy-, eine Aryloxy-, eine niedere Alkylmercapto-, Alkylamino-, Dialkylamino-, eine Hydroxy-, Nitro-, Cyano-, $C_1$-$C_8$-Alkylsulfonamido-, Arylsulfonamido-, $C_1$-$C_8$-Alkylsulfon-, Arylsulfon-, Sulfonsäure-, Carbonsäure-, eine N-Morpholino-, N-Thiomorpholino-, N-pyrrolidino, eine gegebenenfalls N'-alkylierte N-Piperazino-, N-Piperidino-, Phenylamino-, eine gegebenenfalls mit einem niederen Alkyl- oder niederen Alkoxy-Rest substituierte Phenyl-Gruppe, Halogen oder Wasserstoff,

$R'_2$, $R'_4$, $R'_5$, die gleich oder verschieden sein können, jeweils eine niedere Alkyl-, eine niedere Alkoxy-, Nitro-, $C_1$-$C_8$-Alkylsulfonamido-, Arylsulfonamido-, $C_1$-$C_8$-Alkylsulfon-, Arylsulfon-, Sulfon-

säure-, Carbonsäure-, eine niedere Alkylmercapto-Gruppe, Halogen oder Wasserstoff und

X ein stabilisierendes Anion

bedeuten, wobei jeweils 2 benachbarte Reste $R'_1$ bis $R'_5$ zu einem gegebenenfalls durch Halogen, eine $C_1$-$C_6$-Alkyl-, eine $C_1$-$C_6$-Alkoxy-, eine Nitro, Sulfonsäure- oder Carbonsäuregruppe substituierten Benzolring ringgeschlossen sein können, so daß ein Diazoniumsalz der Naphthalinreihe entsteht.

Vorzugsweise stehen in der allgemeinen Formel (V)

| | |
|---|---|
| $R'_1$ | für $C_1$- bis $C_4$-Alkyl, $C_1$-$C_4$-Alkoxy, Hydroxy, Nitro, Halogen oder Wasserstoff, |
| $R'_3$ | für eine $C_1$- bis $C_4$-Alkyl-, $C_1$-$C_4$-Alkoxy-, Aryloxy-, $C_1$-$C_4$-Alkylamino-, $C_1$-$C_4$-Dialkylamino-, Nitro-, $C_1$-$C_4$-Alkylsulfonamido-, Arylsulfonamido-, $C_1$-$C_4$-Alkylsulfon-, Arylsulfon-, N-Morpholino-, N-Pyrrolidino-, Phenylamino- oder Sulfonsäuregruppe oder Wasserstoff und |
| $R'_2$, $R'_4$, $R'_5$, | die gleich oder verschieden sein können, für $C_1$- bis $C_4$-Alkyl-, $C_1$- bis $C_4$-Alkoxy-, $C_1$ -bis $C_4$-Alkylamino-, $C_1$ bis $C_4$-Dialkylamino-, Nitro-, $C_1$- bis $C_4$-Alkylsulfonamido-, Arylsulfonamido- oder Sulfonsäuregruppen, Halogen oder Wasserstoff. |

Jeweils 2 benachbarte Reste $R'_1$ bis $R'_5$ können dabei gegebenenfalls zu einem gegebenenfalls durch Halogen, eine $C_1$ bis $C_4$-Alkyl-, $C_1$- bis $C_4$-Alkoxy-, Nitro-, Halogen- oder Sulfonsäuregruppe substituierten Benzolring ringgeschlossen sein.

Im Rahmen der Formel (V) steht Aryl jeweils für einen gegebenenfalls durch Halogen, eine $C_1$-$C_4$-Alkyl- oder $C_1$-$C_4$-Alkoxygruppe substituierten aromatischen Rest mit 6 bis 12 C-Atomen, vorzugsweise 6 C-Atomen.

Die Diazoniumsalze der allgemeinen Formel (V) sind an sich bekannt oder können nach an sich bekannten Methoden synthetisiert werden (Houben-Weyl, Methoden der organischen Chemie, Band X/3).

Vorzugsweise enthalten die erfindungsgemäßen Mittel zum Nachweis proteolytischer Enzyme und insbesondere der Leukozyten-Enzyme ein geeignetes Puffersystem. Hierfür kommen z. B. Phosphat-, Borat-, Carbonat/Hydrogencarbonat-, Carbonat-, Barbiturat-, Tris-(hydroxymethyl)aminomethan-(=Tris)-, 2-Amino-2-methyl-propandiol-1,3-(=Amediol)- oder Aminosäure-Puffer in Frage, wobei in der Regel pH-Wert und Kapazität so gewählt werden, daß sich in der Meßlösung bzw. auf dem Teststreifen ein pH-Wert von 6 - 10, vorzugsweise von 7 - 9, einstellt.

In manchen Fällen kann es vorteilhaft sein, in den erfindungsgemäßen Mitteln auch Detergentien mitzuverwenden, da hierdurch eine homogenere Farbverteilung und eine intensivere Färbung erzielt werden können. In Trage kommen sowohl kationenaktive als auch anionenaktive Detergentien aber auch amphotere und nichtionogene Detergentien. Als Beispiele hierfür seinen Benzyl-dimethyl-tetradecylammoniumchlorid, Na-Dodecylsulfat, Zephirol, Polyvinylpyrrolidon, Heparinoid und basische Aminosäuren enthaltende Homo- oder Co-Polyaminosäuren [E.Katchalski und M. Sela in: Advances of Protein Chemistry 13, 243 - 492 (1958); C. B. Anfinsen, H. L. Anson, J. T. Edsall und K. Bailey (Hrsg.); Academic Press. Inc. Publishers, New York, N.Y.] sowie sequentielle Polyaminosäuren genannt. Als basische Aminosäuren kommen solche Aminosäuren in Frage, die in den Seitenketten Amino- oder Guanidinogruppen tragen. Es sind dies insbesondere Lysin und Ornithin sowie Arginin aber auch nicht in natürlichen Proteinen vorkommende basische Aminosäuren wie beispielsweise Diaminobuttersäure, Diaminopropionsäure oder Diaminopimelinsäure. In den Polyaminosäuren können die konstituierenden Aminosäuren racemisch oder optisch aktiv in der D- oder L-Form vorliegen. Die Molekulargewichte der Polyaminosäuren betragen 1000 - 2 000 000 und liegen vorzugsweise zwischen 5000 und 500 000. Die Gehalte an basischen Aminosäuren können zwischen 5 und 100 Molprozent betragen, vorzugsweise zwischen 20 und 100 Molprozent, bezogen auf die Polyaminosäure.

Gegebenenfalls können auch Gemische von zwei oder mehr der oben aufgeführten Detergentien eingesetzt werden.

Die Detergentien werden im Flüssigtest vorzugsweise in Konzentrationen von $10^{-4}$ - $10^{-1}$ Gew.-% eingesetzt, besonders bevorzugt zwischen $10^{-4}$ und $10^{-2}$ Gew.-%. In den Imprägnierlösungen beträgt ihr Anteil vorzugsweise 0.01 bis 10 Gew.-%, besonders bevorzugt liegt er bei 0.1 - 5 Gew.-%.

Besonders vorteilhaft ist die Verwendung der obigen Detergentien bei der Bestimmung der Leukozyten mit Hilfe der auf einer festen Phase fixierten Reagentienmischung, da hierdurch eine homogenere Farbverteilung und eine intensivere Färbung erzielt werden können.

Vorzugsweise sind bei den erfindungsgemäßen Mitteln die verschiedenen oben beschriebenen Reagenzien in einen inerten Trägermaterial der an sich bekannten Art inkorporiert, wobei als Trägermatrix besonders bevorzugt poröse Materialien wie insbesondere Filterpapier, aber auch Kunststoffmembranen, Glasfasermatten (US-PS-3 846 247), poröse Keramikstreifen, Kunstfaservliese, schwammartige Materialien (US-PS-3 552 928), Filz, Textilien, Holz, Cellulose oder auch Silicagel in Frage kommen.

Die genannten Trägermaterialien werden zu diesem Zweck mit einer Lösung der oben beschriebenen Reagentien in einem geeigneten leicht entfernbaren Lösungsmittel, z. B. Wasser, Methanol, Ethanol, Aceton, DMF oder DMSO imprägniert. Vorzugsweise geschieht dies in zwei getrennten Schritten: Zunächst wird mit einer wäßrigen Lösung imprägniert, die den Puffer und andere wasserlösliche Zusatzstoffe enthält. Danach wird mit einer Lösung der chromogenen Enzym-Substrate der allgemeinen Formel (V) und des Aktivators imprägniert. Die Imprägnierung kann jedoch auch in einer anderen Reihenfolge bzw. mit einer anderen Zusammensetzung der beiden Imprägnierlösungen durchgeführt werden. Vorzugsweise enthält die Imprägnierlösung oder die zu untersuchende Flüssigkeit das chromogene Substrat und das Diazoniumsalz jeweils in einer Konzentration von $10^{-4}$ bis $10^{-1}$ mol/l, insbesondere $10^{-3}$ bis $10^{-2}$ mol/l, und das Undecanol in einer Konzentration von 0,01 Gew.-% bis 10 Gew.-%, insbesondere von 0,5 Gew.-% bis 5 Gew.-%.

Bei Verwendung von Filterpapier als Matrix können die fertigen Testpapiere als solche verwendet werden oder in an sich bekannter Weise an Griffen angeklebt oder vorzugsweise zwischen Kunststoffen und feinmaschigen Netzwerken z. B. gemäß DE-OS-2 118 455 eingesiegelt werden.

Zur Herstellung filmbeschichteter Teststreifen werden vorzugsweise sämtliche Reagenzien in die Lösung oder Dispersion einer filmbildenden Substanz, wie z. B. Polyvinylester oder Polyamid, eingetragen und homogen vermischt. Das Gemisch wird in dünner Schicht auf einen Kunststoffträger gestrichen und getrocknet. Die so hergestellten filmbeschichteten Teststreifen werden nach dem Trocknen geschnitten und können als solche verwendet oder in an sich bekannter Weise an Griffen angeklebt werden oder z. B. zwischen Kunststoffen und feinmaschigen Netzwerken gemäß DE-OS-2 118 455 eingesiegelt werden.

Ein erfindungsgemäßes diagnostisches Mittel zum Nachweis esterolytischer und/oder proteolytischer Enzyme, insbesondere der Leukozyten-Enzyme, in Form von Pulvermischungen oder Reagenztabletten läßt sich herstellen, indem die oben angeführten Bestandteile des Testmittels mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Pulvermischungen oder Reagenztabletten weisen z. B. ein Endgewicht von ungefähr 50 - 200 mg, vorzugsweise 50 - 80 mg, auf.

Zur Herstellung von Lyophilisaten im Gesamtgewicht von jeweils etwa 5 - 20 mg, vorzugsweise etwa 10 mg, wird eine Lösung gefriergetrocknet, die neben sämtlichen für den Test benötigten Reagenzien übliche Gerüstbildner, wie z. B. Polyvinylpyrrolidon, und evtl. weitere Füllstoffe, wie z. B. Mannit, Sorbit oder Xylit, enthält.

Ein erfindungsgemäßes diagnostisches Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z. B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit esterolytischer und/oder proteolytischer Enzyme, insbesondere der Leukozyten-Enzyme, rasch und einfach über eine Farbbildung nachzuweisen, die visuell oder photometrisch, z. B. remissions-photometrisch oder in der Küvette, gemessen werden kann. Da die Aktivität der Leukozyten-Enzyme pro Zelle als eine im wesentlichen konstante Größe angesehen werden kann, läßt sich aus der Intensität der Farbbildung die Leukozytenkonzentration der untersuchten Körperflüssigkeit ermitteln. Dabei werden mit dem erfindungsgemäßen diagnostischen Mittel sowohl intakte als auch lysierte Leukozyten erfaßt, da die Aktivität der Leukozyten-Enzyme auch nach der Lyse der Leukozyten voll erhalten bleibt. Ein Lysefehler tritt folglich nicht auf.

Die nachfolgenden Beispiele dienen zur Erläuterung der vorliegenden Erfindung. Wenn nicht anders vermerkt, sind Mengenangaben als Gewichtsteile oder Gewichtsprozente zu verstehen.

**Allgemeine Arbeitsvorschrift**

Zu 2.25 ml des betreffenden Puffers fügte man je nach Substrat 50 - 250 µl N-Methylpyrrolidon als Lösungsvermittler und ergänzte das Volumen der Lösung mit Puffer auf 2.5 ml. Dann wurden 5 µl einer Lösung von 5 - 100 mg n-Undecanol in 1 ml Methylpyrrolidon sowie 5 µl einer Lösung von 2 mg Natriumdodecylsulfat (SDS) bzw. 4 mg der anderen Detergentien in 1 ml Wasser oder N-Methylpyrrolidon zu der Reaktionsmischung zugegeben. Nach gutem Durchmischen wurden 5 µl einer $10^{-1}$ molaren Substratlösung in N-Methylpyrrolidon, Dimethylformamid oder Dimethylsulfoxid in die Reaktionslösung gegeben und nach der Zugabe der Leukozytensuspension der Extinktionsanstieg bei der angegebenen Wellenlänge kontinuierlich verfolgt. Die Spontanhydrolyse des Substrats wird in einem Paralellansatz ohne Leukozytenzugabe anhand des Extinktionsanstiegs bestimmt.

Für die Bestimmung der Umsetzungsgeschwindigkeit wird der in der Enzymreaktion erhaltene Extinktionsanstieg um den für die Spontanhydrolyse ermittelten Betrag vermindert. Absolutwerte für die Substratspaltung (Mol. $Min^{-1}$) können aus den Extinktionsdifferenzen mit Hilfe der molaren Extinktionskoeffizienten errechnet werden.

**Beispiel 1**

Filtrierpapier (z. B. Eaton und Dikeman 205) wird nacheinander mit den folgenden Lösungen imprägniert und dann bei 60°C getrocknet.

<u>Lösung 1:</u> 0.1 H Tris-(hydroxymethyl)-aminomethan-Salzsäure-Puffer (pH = 8.8), enthaltend 3 % Polyvinylpyrrolidon.

Lösung 2: $7.5 \times 10^{-3}$ mol/l N-Tosyl-L-alanin-5-hydroxy-1,2-benzisothiazolylester, $10^{-2}$ mol/l 2,4-Dimethoxybenzoldiazonium-tetrafluoroborat und 3.5 g n-Undecanol/l in wasserfreiem Aceton.

Man erhält ein schwach gelb gefärbtes Testpapier, das sich beim Eintauchen in leukozytenhaltige Urine rotbraun verfärbt.

**Beispiel 2**

Beschleunigende Wirkung von Alkanolen auf die enzymatische Spaltung von N-Tosyl-L-alanin-indoxylester mit Leukozyten in 0.1 M Tris-(hydroxymethyl)-aminomethan-Salzsäure-Puffer pH 8.8 unter Zusatz von SDS bzw. Polylysin mit einem mittleren Molekulargewicht von 14 000 (Messung bei 360 nm).

**Tabelle 1**

| Aktivator | Detergens | µg/Test | relative Spaltungsgeschwindigkeit |
|---|---|---|---|
| - | - | - | 0.65 |
| n-Heptanol | SDS | 10 | 0.65 |
| n-Octanol | SDS | 10 | 0.75 |
| n-Nonanol | SDS | 10 | 0.85 |
| n-Decanol | SDS | 10 | 1 |
| n-Undecanol | SDS | 10 | 1.85 |
| n-Dodecanol | SDS | 10 | 0.55 |
| n-Tridecanol | SDS | 10 | 0.75 |
| n-Decanol | Poly-L-Lys | 20 | 3.2 |
| n-Undecanol | " | 20 | 6.5 |

**Beispiel 3**

Konzentrationsabhängigkeit der beschleunigenden Wirkung von n-Undecanol auf die Spaltung von N-Tosyl-L-alanin-indoxylester mit Leukozyten in Gegenwart von 50 µl. N-Methylpyrrolidon/Testansatz und 10 µg/Test SDS in 0.1 M Tris- (hydroxymethyl) -aminomethan-Salzsäurepuffer (pH 8.8).

(Messung bei 360 nm)

**Tabelle 2**

| µl Undecanol in der Testlösung | rel.Geschwindigk. der Enzymreaktion, bezogen auf Decanol (0,125 µl/Test) = 1 |
|---|---|
| 0,025 | 0,65 |
| 0,075 | 1,5 |
| 0,125 | 1,75 |
| 0,2 | 2,1 |
| 0,25 | 3,2 |
| 0,5 | 3,1 |

**Beispiel 4**

Beschleunigung der Spaltung von N-Tosyl-L-alanin-5-hydroxy-1,2-benzisothiazolylester mit Leukozyten in 0.1 Tris-(hydroxymethyl)-aminomethan-Salzsäurepuffer (pH 8.4) in Gegenwart von 100 µl N-Methylpyrrolidon durch Zusätze von Aktivatoren und Detergentien.

(Messung bei 325 nm).

**Tabelle 3**

| Aktivator | µg/Test | Detergens | µg/Test | relative Spaltungsgeschwindigkeit |
|---|---|---|---|---|
| - | - | - | - | 0,5 |
| Decanol | 125 | - | - | 1.9 |
| " | " | SDS | 10 | 1.0 |

| | | | | |
|---|---|---|---|---|
| " | " | Poly-L-Lys (14000) | 20 | 4.25 |
| " | " | Poly-L-Orn (30000) | 20 | 3.8 |
| Undecanol | " | - | - | 3.1 |
| " | " | SDS | 10 | 2.5 |
| " | " | Poly-L-Lys (14000) | 20 | 8.2 |
| " | " | Poly-L-Orn (30000) | 20 | 7.4 |

In Klammern sind die mittleren Molekulargewichte der Polyaminosäuren (Zahlenmittel) angegeben.

**Beispiel 5**

Beschleunigung der Spaltung von N-Tosyl-L-alanin-3-hydroxy-5-phenylpyrrolester mit Leukozyten in 0.1 M Tris-(hydroxymethyl)-aminomethan-Salzsäurepuffer (pH 8.8) in Gegenwart von 250 µl N-Methylpyrrolidon durch Zusätze von Aktivatoren und Detergentien.

(Messung bei 330 nm)

**Tabelle 4**

| Aktivator | µg/Test | Detergens | µg/Test | relative Spaltungsgeschwindigkeit |
|---|---|---|---|---|
| Decanol | 125 | SDS | 10 | 1.0 |
| " | 125 | Zephirol | 20 | 1.27 |
| Undecanol | 125 | SDS | 10 | 3.3 |
| " | 125 | Zephirol | 20 | 2.6 |

Zephirol wurde als ca. 50 %-ige wäßrige Lösung eingesetzt.

**Beispiel 6**

Beschleunigung der Spaltung von N-Tosyl-L-alanin-3-hydroxy-5-phenylpyrrolester mit Leukozyten in diversen 0,1 M. Puffern (pH 8.4) unter Zusatz von 250 µl N-Methylpyrrolidon und Aktivatoren sowie Detergentien.

(Messung bei 330 nm)

**Tabelle 5**

| Puffer | Aktivator 0.125 µl bzw. 125 µg je Test | Detergens | µg/Test | relative Spaltungsgeschwindigkeit |
|---|---|---|---|---|
| Tris[1)] | - | - | - | 0.8 |
| | - | SDS | 10 | 0.8 |
| | n-Nonanol | SDS | " | 0.6 |
| | n-Decanol | - | - | 0.9 |
| | " | Poly-L-arg (40000) | 20 | 3.7 |
| | " | BDTA | " | 1.05 |
| | " | SDS | 10 | 1 |
| | n-Undecanol | - | - | 1.7 |
| | " | Poly-L-Arg (40000) | 20 | 6.55 |
| | " | BDTA | " | 3.55 |
| | " | SDS | 10 | 2.1 |
| Borat | - | - | - | 2.35 |
| | - | SDS | 10 | 2.8 |
| | n-Nonanol | " | " | 3.0 |
| | n-Decanol | - | - | 4.9 |
| | " | Poly-DL-Lys (37000) | 20 | 3.15 |
| | " | SDS | 10 | 9.0 |
| | n-Undecanol | - | - | 5.6 |
| | " | SDS | 10 | 15.2 |
| | n-Dodecanol | " | " | 9.0 |
| Phosphat | - | - | - | 1.55 |

| | | | | |
|---|---|---|---|---|
| | n-Decanol | SDS | 10 | 12.0 |
| | n-Undecanol | " | 10 | 17.3 |
| Carbonat- | - | - | - | 1.5 |
| Hydrogen- | n-Decanol | SDS | 10 | 5.6 |
| carbonat | n-Undecanol | SDS | 10 | 6.6 |

Tris = Tris-(hydroxymethyl)-aminomethan-Salzsäurepuffer
BDTA = Benzyl-dimethyl-tetradecylamin-hydrochlorid

In Klammern sind die mittleren Molekulargewichte der Polyaminosäuren angegeben.

**Allgemeine Arbeitsvorschrift zur Herstellung der N-Tosyl-L-alanylester:**

Die Ester wurden jeweils durch Umsetzung von N-Tosyl-L-alanylchlorid mit den Phenolen in absolutem Methylethylketon oder absolutem Toluol in Gegenwart von gepulvertem Kaliumcarbonat hergestellt. Nach 6 bis 12-stündigem Rühren bei ca. 55°C waren zwischen 40 und 70 % des Phenols umgesetzt. Das Molverhältnis Phenol : $K_2CO_3$ Säurechlorid betrug zumeist 1 : 1,5 : 1,5. Der pH-Wert lag während der gesamten Reaktionszeit um 7. Zur Aufarbeitung wurde das Kaliumcarbonat bei 50°C abfiltriert und danach das Lösungsmittel im Vakuum abdestilliert. Die Reinigung erfolgte über Säulenchromatographie mit Kieselgel (Laufmittel Petrolether: Aceton = ca. 9 : 1) und anschließendes Umkristallisieren.

**L-p-Tosylalanin**

Literatur: E. Fischer u. W. Lipschitz, B. 48, 362 (1915).

83,7 g (0,93 Mol) L-Alanin werden in 465 ml ca. 2N-Natronlauge gelöst. Die Lösung wird bei 70 - 72°C portionsweise mit 186 g (0,976 Mol) p-Toluolsulfochlorid in 20 Minuten versetzt. Während der Zugabe des Sulfochlorids wird die Reaktionsmischung durch einen automatischen Titrator mit ca. 2N-Natronlauge bei pH 10 gehalten; dabei werden 560 ml 2N-Natronlauge verbraucht. Wenn sich der pH der Reaktionsmischung nicht mehr ändert, kühlt man diese auf 15 - 5°C ab und stellt mit 37 %-iger Salzsäure auf pH 3 ein. Das abgeschiedene Produkt wird abgesaugt, den feuchten Filterkuchen löst man aus 2350 ml Wasser um.
Ausbeute: 185,5 g (82 % der Theorie) L-p-Tosylalanin vom m.p. 132 - 135°C.

**p-Tosyl-L-alanylchlorid**

158,1 g (0,65 Mol) L-p-Tosylalanin werden in 350 ml Thionylchlorid bei 40°C gerührt, bis eine klare Lösung entstanden ist. Dann destilliert man das überschüssige Thionylchlorid im Wasserstrahlvakuum ab. Den Kolbenrückstand nimmt man in 300 ml destilliertem Toluol auf. Man erhält eine klare, schwach gelbliche Lösung, die in 900 ml gerührtes Waschbenzin eingegossen wird. Das Säurechlorid fällt aus. Es wird am nächsten Tage abgesaugt, mit Leichtbenzin gewaschen und in einem Vakuumexsiccator über Calciumchlorid /Kaliumhydroxyd getrocknet.
Ausbeute: 155 g (91 % der Theorie) an fast farblosen Kristallen vom m.p. 81 - 83°C.

**Patentansprüche**

1. Mittel zum Nachweis esterolytischer und/oder proteolytischer Enzyme, enthaltend

(a) einen Aminosäure- oder Peptidester eines Phenols als chromogenes Enzymsubstrat und
(b) einen Alkohol als die enzymatische Spaltung der Aminosäure- bzw. Peptidesterbindung von Komponente (a) beschleunigende Substanz,

dadurch gekennzeichnet, daß es als beschleunigende Substanz n-Undecanol enthält.
2. Mittel nach Anspruch 1, dadurch gekennzeichnet, daß die Reagenzien in einem inerten Trägermaterial, vorzugsweise in Form eines Teststreifens, inkorporiert sind.
3. Verfahren zum Nachweis von esterolytischen und/oder proteolytischen Enzymen in flüssigen Proben,

insbesondere Körperflüssigkeiten, dadurch gekennzeichnet, daß man die Probe mit einem Mittel nach Anspruch 1 oder 2 in Kontakt bringt und die auftretende Farbreaktion bestimmt.

4. Verwendung von n-Undecanol als Aktivator beim Nachweis von esterolytischen und/oder proteolytischen Enzymen durch Spaltung von chromogenen Aminosäureestern oder Peptidestern von Phenolen.

**Claims**

1. Agent for the detection of esterolytic and/or proteolytic enzymes, containing (a) an aminoacid ester or peptide ester of a phenol, as the chromogenic enzyme substrate, and (b) an alcohol as the substance which accelerates the enzymatic cleavage of the aminoacid ester bond or peptide ester bond of component (a), characterised in that it contains n-undecanol as the accelerating substance.

2. Agent according to claim 1, characterised in that the reagents are incorporated in an inert carrier, preferably in the form of a test strip.

3. Process for the detection of esterolytic and/or proteolytic enzymes in liquid samples, in particular body fluids, characterised in that the sample is brought into contact with an agent according to claim 1 or 2 and the colour reaction which occurs is determined.

4. Use of n-undecanol as an activator in the detection of esterolytic and/or proteolytic enzymes by cleavage of chromogenic aminoacid esters or peptide esters of phenols.

**Revendications**

1. Agent pour la détection d'enzymes estérolytiques et/ou protéolytiques, contenant

a) un ester d'aminoacide ou de peptide d'un phénol comme substrat d'enzyme chromogène et
b) un alcool comme substance accélérant la dissociation enzymatique de la liaison ester d'aminoacide ou de peptide du composant (a),

caractérisé en ce qu'il contient comme substance accélératrice du n-undécanol.

2. Agent selon la revendication 1, caractérisé en ce que les réactifs sont incorporés dans un matériau de support inerte, de préférence sous forme d'une bande d'essai.

3. Procédé pour la détection d'enzymes estérolytiques et/ou protéolytiques dans des échantillons liquides, en particulier des liquides de l'organisme, caractérisé en ce que l'on met en contact l'échantillon avec un agent selon la revendication 1 ou 2 et on détermine la réaction colorée qui se produit.

4. Utilisation du n-undécanol comme activateur dans la détection d'enzymes estérolytiques et/ou protéolytiques par dissociation d'esters phénoliques chromogènes d'aminoacides ou de peptides.